# EUROPEAN PATENT APPLICATION

(11) **EP 1 723 961 A2**
(43) Date of publication of application: **22.11.2006**
(21) Application number: 06113627.1
(22) Date of filing: 08.05.2006
(51) Int. Cl.: A61K 38/18, A61P 19/08

(54) **Pharmaceutical composition comprising at least one bone morphogenetic protein (BMP)**

(30) Priority: 10.05.2005 US 125847
(71) Applicant: University of Zürich, 8006 Zürich (CH)
(72) Inventor: Weber, Franz, 78224 Singen (DE)
(74) Representative: Lax, Monica Ingeborg

(57) **Abstract**

The present invention relates to pharmaceutical compositions containing at least one bone morphogenetic protein (BMP) and a plasticizer in a pharmaceutically acceptable carrier, such as in a biodegradable polymer, in amounts providing a synergistic bone forming and bone healing effect. The present invention further relates to methods of treating orthopedic and dental, including periodontal, diseases by simultaneously administering to a subject in a need of such treatment at least one bone morphogenetic protein (BMP) and at least one plasticizer optionally in a pharmaceutically acceptable carrier, in amounts providing a synergistic bone forming effect.

## Description

### FIELD OF THE INVENTION

The present invention relates to pharmaceutical compositions containing at least one bone morphogenetic protein (BMP) and a plasticizer in a pharmaceutically acceptable carrier, such as in a biodegradable polymer, in amounts providing a synergistic bone forming and bone healing effect. The present invention further relates to methods of treating orthopedic and dental, including periodontal, diseases by simultaneously administering to a subject in a need of such treatment at least one bone morphogenetic protein (BMP) and at least one plasticizer optionally in a pharmaceutically acceptable carrier, in amounts providing a synergistic bone forming effect.

### BACKGROUND OF THE INVENTION

In the last decade guided bone regeneration (GBR) has been introduced as a predictable and effective method for enhancing bone healing, and in particular in conjunction with the placement of dental implants GBR is a clinically well-documented and successful procedure [Dahlin, C., et al., Int. J. Periodontics Restorative Dent. 11 (1991) 273-281; Hämmerle, C. H. and Karring, T., Periodontol. 2000 17 (1998) 151-175; Nyman, S. R. and Lang, N. P. Periodontol. 2000 4 (1994) 109-118]. In GBR, a membrane serves as a barrier for the connective tissue and maintains an open space for the time bone needs to fill it up.

An acceleration of this bone filling process can be achieved by the principle of osteoconduction, if the empty space is filled with porous materials, which serve as a scaffold for the newly formed bone [Reddi, H., Cytokine & Growth Factor Reviews 8 (1997) 11-20]. Alternatively, bone repair can be accelerated by osteoinduction, which involves the application of appropriate growth factors capable to differentiate mesenchymal stem cells to osteoblasts [Wozney, J. M. and Rosen, V., Clin. Orthop. Rel. Res. 346 (1998) 26-37].

The most useful growth factors in osteoinduction are bone morphogenetic proteins (BMPs), which are differentiation factors and have been isolated based on their ability to induce bone formation [Wozney, J. M., et al., Science 242 (1988) 1528-1534]. They build the BMP-family with more than thirty members belonging to the TGF-β-super-family. The BMP-family is divided to subfamilies including the BMPs, including BMP-2 and BMP-4, osteogenic proteins (OPs), including OP-1 or BMP-7, OP-2 or BMP-8, BMP-5, BMP-6 or Vgr-1, cartilage-derived morphogenetic proteins (CDMPs), including CDMP-1 or BMP-14 or GDF-5, growth/differentiation factors (GDFs), including GDF-1, GDF-3, GDF-8, GDF-9, GDF-11 or BMP-11, GDF-12 and GDF-14, and other subfamilies, including BMP-3 or osteogenin, BMP-9 or GDF-2, and BMP10 (Reddi *et al.,*1997, *supra*).

Especially in animal models the BMPs have proved to be powerful inducers of bone formation and repair. However, due to the instant degradation of the BMPs upon contact with body fluids and the strong morphogenetic action of the BMPs, un-physiologically high doses of the BMPs are needed for the osteoinductive bioactivity [Weber, F. E., et al., Int. J. Oral Maxillofac. Surg. 31 (2002) 60-65; Rose, F. R. A. and Oreffo, R. O. C. Biochem. Biophys. Res. Com. 292 (2002) 1-7]. Topical administration routes must be used, which makes the choice of the carrier system critical, and suitable carrier systems are currently not commercially available. Since the BMPs are usually produced with recombinant techniques and thus are expensive and available only in limited amounts, the BMPs, despite the acknowledged effect, have had only a marginal impact on the medical treatment of patients.

Biodegradable (also referred to as resorbable, absorbable, and erodible) polymers are at present the material of choice for controlled-release systems, including those implantable into the patients to be treated due to their transient foreign-body reactions and their ability to regenerate tissues (see, for instance, The Biomedical Engineering Handbook, Bronzino, J.D., Ed., CRC Press, Boca Raton, 2000, chapter 41, pages 41-1 to 41-22). The most studied bioderadable polymers include poly(lactic acid)s (PLA), poly(lactide-co-glycolide)-poly(ethyleneglycol) (PLG-PEG) copolymers, PLG-PGA copolymers, and like. However, synthetic biodegradable polymers are rigid and lack the flexibility needed to be applicable for instance in GBR, especially in dental GBR.

US patent publication 2003-0104029 A1 discloses that the rigidity of biodegradable polymers can be softened and their flexibility increased by a treatment with a known plasticizer, N-methyl-2-pyrrolidone (NMP), to an extent that allows their use in GBR. Additionally, it is disclosed that NMP itself has an unexpected bone formation inducing effect. In a co-pending US patent application entitled "Resorbable Polymer Composition, Implant and Method of Making Implant" filed on May 10, 2005, Serial No. XX/XXX,XXX, incorporated herein by reference, the Applicant shows that the same applies to another known plasticizer, triacetin and closely related compounds thereto.

New, alternative approaches in the utilization of the BMPs as well as in the application of biodegradable polymers as carriers in medicine are continuously needed.

The object of the present invention is to meet this need and provide new means to overcome the disadvantages and the drawbacks described above.

An object of the present invention is thus to provide novel means for the utilization of the bone forming inductive activity of the BMPs in the treatment of patients in dentistry, including periodontology, and in other fields in medicine, such as orthopedics.

Another object of the invention is to provide means, which afford a dose reduction of the BMPs, especially recombinant BMPs, thereby allowing a safe and cost effective use of the BMPs in the treatment of patients in dentistry, including periodontics, and in other fields in medicine, such as orthopedics.

Still a further object of the invention is to provide new means for the use of guided bone regeneration (GBR).

Yet another object of the invention is to provide new means for the use of biodegradable membranes in GBR, whereby the second surgical stage can be avoided.

The objects of the present invention are met by provision of pharmaceutical compositions, in which the bone morphogenetic properties of the BMPs together with the bone formation inducing properties of glycerol mono-, di-, and triesters with carboxylic acids having 1 to 6 carbon atoms, including glyceryl triacetate and glyceryl diacetate, i.e., triacetin and diacetin, respectively, are utilized in synergistic manner, the compositions being useful in the treatment of dental and periodontal diseases, including the integration of dental implants and the filling of tooth sockets following extraction, specifically via guided bone regeneration (GBR). In the present context, the common terms "triacetin" and "diacetin" are alternatively used of glyceryl triacetate and glyceryl diacetate, respectively.

The objects of the present invention are further met by provision of a method of treating a subject in a need of induction of bone formation, in which the bone morphogenetic properties of the BMPs together with the bone formation inducing properties of glycerol mono-, di-, and triesters with carboxylic acids having 1 to 6 carbon atoms, including glyceryl triacetate and glyceryl diacetate, i.e., triacetin and diacetin, respectively, and optionally biodegradable polymers are administered in a synergistic bone forming manner, to treat dental and periodontal diseases, including the integration of dental implants and the filling of tooth sockets following extraction, and to treat orthopedic diseases and failures, in which the enhancement of fracture healing and the augmentation of bone is desired, such as in the alveolar ridge augmentation, in the sinus floor elevation, and in the healing of non-unions, including improvement of the recovery of patients having a surgical bone operation or accidental bone fractures.

US Patent 6,440,444 B1 discloses a load-bearing osteoimplant composition comprising a shaped composition of bone particles with a given bulk density and a given wet compressive strength. Mono- and diesters of glycerol, i.e., monoacetin and diacetin, are named as wetting agents and as plasticizers optionally used in the composition, however no specific example of their use is given.

US Patents 5,510,396 and 5,314,476 disclose a process for preparing an osteogenic composition and a flowable osteogenic composition, respectively. These compositions contain demineralized bone particles and a biocompatible carrier, such as a liquid polyhydroxy compound or a derivative thereof. Monoacetin and diacetin are named but not exemplified.

US Patent 6,616,698 discloses an implantable, biocompatible osteogenic bone graft comprising a coherent mass of bone particles possessing at least a zone of impermeability to soft tissue in-growth. In the preparation of this implant, a slurry or a paste of bone particles is made in a biocompatible liquid, such as esters of polyhydroxy compounds including monoacetin and diacetin.

US Patent Publication US20020035401 A1 discloses an osteogenic osteoimplant in the form of a flexible sheet comprising bone particles and optionally a biocompatible component, such as a biocompatible fluid carrier, including monoacetin and diacetin. These polyhydroxy compounds are listed as plasticizers but not used in the examples.

However, in none of these prior art publications there is any mention or suggestion of a possible bone formation inducing or bone healing activity of these polyhydroxy compounds and the implants or compositions always contain bone particles. Additionally, the compositions always contain bone particles.

US-patent publication 20040152627 A1 discloses a pharmaceutical composition comprising at least one bone morphogenetic protein (BMP) and at least one pyrrolidone, for instance 1-methyl-2-pyrrolidone (NMP) or 1-ethyl-2-pyrrolidone (NEP), in a pharmaceutically acceptable carrier, said BMP(s) and said pyrrolidone(s) showing a synergetic therapeutic effect on bone formation. Pyrrolidone-type plasticizer used in this publication differ essentially in structure from glycerol derivatives, such as glyceryl triacetate and glyceryl diacetate.

### SHORT DESCRIPTION OF THE INVENTION

Surprisingly it has now been discovered that administration of the BMPs in combination of glycerol mono-, di-, and triesters with carboxylic acids having 1 to 6 carbon atoms, including glyceryl triacetate and glyceryl diacetate, i.e., triacetin and diacetin, respectively, in particular glyceryl triacetate, i.e., triacetin, alone or with biodegradable implants, especially membranes, which have been pre-treated with said glycerol esters, enhances bone formation in a synergistic manner to an extent, which could not have been expected on the basis of separate individual bone formation inducing activities of the BMPs or triacetin, or a combination of triacetin and a biodegradable polymer reported in the prior art.

The present invention relates to a pharmaceutical composition comprising at least one bone morphogenetic protein (BMP) and at least one glycerol mono-, di-, and triester with a carboxylic acid having 1 to 6 carbon atoms, including glyceryl triacetate and glyceryl diacetate, i.e., triacetin and diacetin, respectively, optionally in a pharmaceutically acceptable carrier, wherein said rBMP and said glycerol mono-, di-, and triesters are present in amounts providing a synergetic bone forming effect.

In a preferred embodiment of the invention recombinant bone morphogenetic proteins (rBMPa) are used.

In another embodiment of the invention the pharmaceutically acceptable carrier is a biodegradable polymer.

In another embodiment of the invention the pharmaceutically acceptable carrier is a biodegradable polymer, which has been pre-treated with glycerol mono-, di-, and triesters with carboxylic acids having 1 to 6 carbon atoms, including glyceryl triacetate and glyceryl diacetate, i.e., triacetin and diacetin, respectively, in particular glyceryl triacetate, i.e., triacetin.

One example of an embodiment of the present invention is a pharmaceutical composition comprising at least one recombinant bone morphogenetic protein (rBMP), glyceryl triacetate and a biodegradable polymer, wherein said rBMP, said glyceryl triacetate and said biodegradable polymer are present in amounts providing a synergistic therapeutic effect on bone formation.

Another example of an embodiment of the present invention is a pharmaceutical composition comprising at least one recombinant bone morphogenetic protein (rBMP), glyceryl triacetate and a biodegradable polymer, which has been pretreated with glyceryl triacetate, wherein said rBMP, said glyceryl triacetate and said pretreated biodegradable polymer are present in amounts providing a synergetic therapeutic effect on bone formation.

The present invention further relates to a method of treating a subject in a need of induction of bone formation, the method comprising a simultaneous administration of at least one recombinant bone morphogenetic protein (rBMP) and at least one glycerol mono-, di-, and triester with a carboxylic acid having 1 to 6 carbon atoms, preferably glyceryl triacetate and glyceryl diacetate, i.e., triacetin and diacetin, respectively, and most preferably glyceryl triacetate, optionally in a pharmaceutically acceptable carrier, wherein said rBMP(s) and said glycerol mono-, di-, and triester(s) are present in amounts providing a synergetic therapeutic effect on bone formation, to said subject.

### DRAWINGS

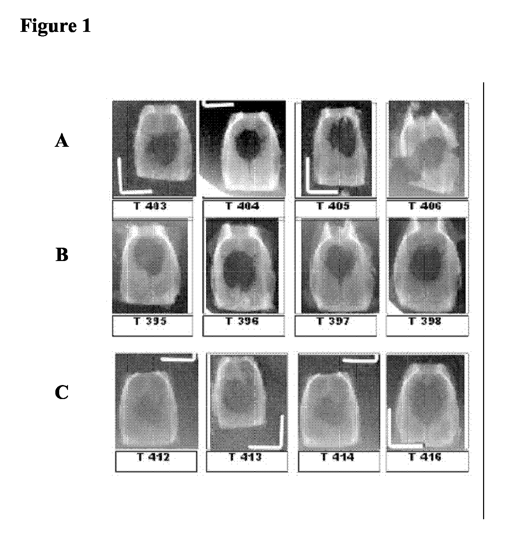

Figure 1 illustrates the enhanced healing of a critical size defect created in the calvarial bone of rats by guided bone regeneration and triacetin: 8 mm (in diameter) defects in the calvarial bone were treated by 2 different membranes (E1M-11®; Fig. 1B), and E1M-11® treated with triacetin; Fig. 1C) or left untreated (control; Fig. 1A). Five weeks after the operation the bone regeneration in the defect was determined. The radiographs of the calvarial bones taken after the sacrifice of the animals are shown.

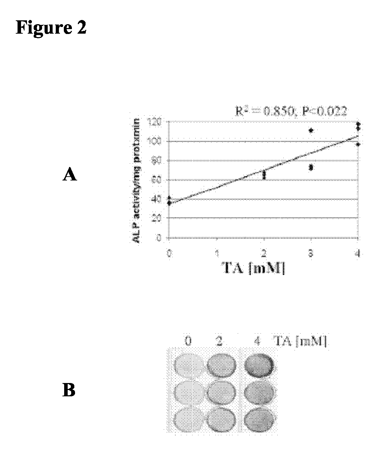

Figure 2 shows the triacetin induced increase of maturation of preosteoblastic cell line (MC3T3-E1) *in vitro.* Fig. 2A: MC3T3-E1 cells treated with increasing amounts of triacetin and the maturation determined after 6 days by alkaline phosphatase activity. Fig. 2B: Mineralization by MC3T3-E1 cells seeded on a 6-well plate visualized by Alizarin S-staining. The MC3T3-E1 cells were cultured for 4 weeks in the presence of 5 mM glycerol phosphate. From the left: no triacetin was added, 2.0 mM triacetin was added, 4.0 mM triacetin.

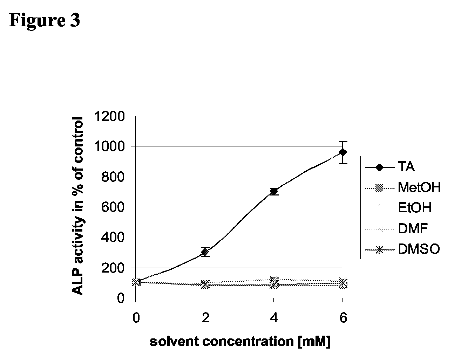

Figure 3 shows the effect of different solvents in comparison with triacetin on the alkaline phosphate activity of preosteoblastic cells.

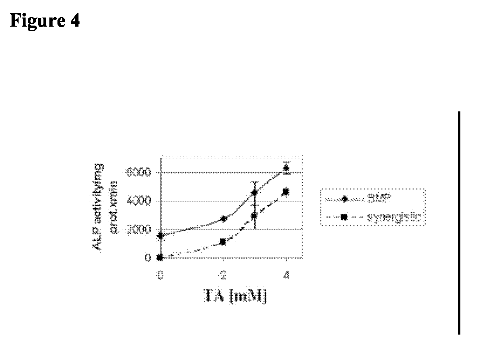

Figure 4 shows the synergistic effect of triacetin and rhBMP-2 on the maturation of MC3T3-E1 cells determined by alkaline phosphatase activity. MC3T3-E1 cells were treated with triacetin in the presence or absence of 1 µg rhBMP-2/ml of the medium. For the calculation of the synergistic effect, the ALP activity induced by rhBMP treatment alone and the ALP activity induced by triacetin treatment alone was subtracted from the ALP activity achieved in the presence of both rhBMP-2 and triacetin. SD is indicated in the graph.

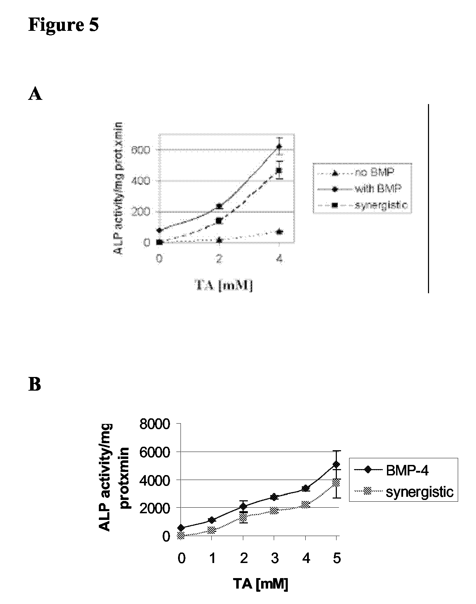

Figure 5 shows the synergistic effect of triacetin and rhBMP-4 on the maturation of MC3T3-E1 cells determined by alkaline phosphatase activity. MC3T3-E1 cells were treated with triacetin in the presence or absence of 60 ng rhBMP-4/ml of the medium. For the calculation of the synergistic effect the ALP activity induced by BMP treatment alone and the ALP activity induced by triacetin treatment alone was subtracted from the ALP activity achieved in the presence of both rhBMP-4 and triacetin. SD is indicated in the graph.

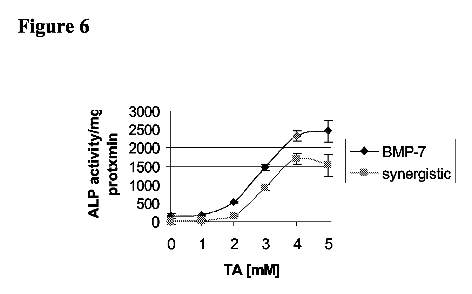

Figure 6 shows the synergistic effect of triacetin and rhBMP-7 on the maturation of MC3T3-E1 cells determined by alkaline phosphatase activity. MC3T3-E1 cells were treated with triacetin in the presence or absence of 60 ng rhBMP-7/ml of the medium. For the calculation of the synergistic effect the ALP activity induced by BMP treatment alone and the ALP activity induced by NMP treatment alone was subtracted from the ALP activity achieved in the presence of both rhBMP-7 and triacetin. SD is indicated in the graph.

### DETAILED DESCRIPTION OF THE INVENTION

The present invention is based on the Applicant's further studies to reveal the mechanisms, how triacetin is capable to enhance bone regeneration. Triacetin-treated membranes, composed of a synthetic biodegradable polymer, namely polylactide/glycolide (PLGA), which membranes are too ridged for use in GBR, show better results in *in vivo* bone regeneration than untreated PLGA-membranes, when softened with a plasticizer, triacetin, both *in vivo* in rats and *in vitro* in a BMP-producing cell line and *in vitro* in a non-BMP-producing cell line (Fig. 2). The latter suggests an improvement in the self-healing capability by increasing the bioactivity of autologous BMPs. However, the use of other solvents/plasticizer conventionally used in the preparations of implants in the art did not induce bone regeneration on the alkaline phosphate activity of preosteoblastic cells (Fig. 3).

The critical size calvarial defect model was chosen as the animal model, because it is the most selective model for bone regeneration due to the poor blood supply and the membranous (cortical) structure of the bone, which has an adverse effect on bone healing. The size of the defect was critical size and would not heal during the live time of the animal. In this animal model two membranes E1M-11® and E1M-11® treated with triacetin (E1M-11®-triacetin) were tested and only the E1M-11®-triacetin improve bone regeneration (Fig. 1).

These results suggest that triacetin accelerates the signal transduction via BMP-receptors or acts downstream the receptor level. This interpretation was supported by *in vitro* data derived from a preosteoblastic cell line (MC3T3-E1), where the application of low triacetin doses enhanced their maturation (Fig. 2). This cell line is known to secrete BMP, to deposit it in the extracellular matrix and to mature by the action of extracellular BMP acting exclusively on the BMP signal transduction via Smads [Suzawa, M., et al., Endocrin. 140 (1999) 2125-2133]. The same signal transduction pathway is also present in C3H10T1/2 cells [Katagiri, T., et al., Biochem. Biophys. Res. Com. 172 (1990) 295-300], where even triacetin treatment alone but especially in combination with rhBMPs lead to an increase in alkaline phosphatase activity. Therefore at least one site of triacetin action is downstream the BMP receptor level, accelerating the effect of autologous and rhBMP. Even without added BMPs triacetin is able to increase alkaline phosphatase activity in C3H10T1/2 cells pointing to osteoinductive effects.

In GBR the combination of a synthetic biodegradable polymer membrane, such as a PLGA membrane, treated with triacetin expands the function of the membrane from a purely mechanical separator of connective tissue from open space generated for bone in-growth to a delivery device for triacetin. By this combination GBR is directly linked to osteoinduction, because to obtain a healing of a critical size defect the application of osteoinductive factors like BMPs is needed. By the acceleration of the bioactivity/bioavailability of autologous BMP *in vivo* in the presence of triacetin, the natural concentration of autologous BMP is sufficient to heal a critical size defect, which under normal conditions would need the application of extra rhBMP.

Therefore the use of triacetin in this model is also a good example for enforcing the self-healing process, in this case via autologous BMP.

These results encouraged the Applicant to further studies on the effect of triacetin on added BMP, with the expectations that the amount of external rBMP needed in the clinical applications could be reduced.

Surprisingly, however, in further studies it was discovered that an administration of the BMPs in combination with triacetin enhances bone formation to an unexpected extent, which could not have been foreseen on the basis of separate individual bone formation inducing activities of the rBMPs or triacetin reported in the prior art (Figs. 4-6). In other words, an administration of the rBMPs in combination with triacetin provides a synergistic bone formation inducing effect.

This affords advantages in terms of smaller amounts of the material needed for the desired effect, which is of great importance in view of the laborious and expensive production of the rBMPs. Also, a risk of side effects decreases significantly, when smaller amounts of foreign material can be used. In all, the invention enables for the first time a cost-effective therapeutic utilization of the bone inducing potential of the BMPs, especially the rBMPs.

For the present purposes, the terms BMP or rBMP refer to a member of the BMP family as a dimeric natural or recombinant product, respectively. Thus, for example, the term rBMP covers the members of the BMP subfamily, such as BMP-2 and BMP-4, the members of the OP subfamily, such as OP-1 (or BMP-7) OP-2 (or BMP-8), BMP-5, BMP-6 (or Vgr-1), the members of the CDMP subfamily, such as CDMP-1 (or BMP-14 or GDF-5), the members of the GDF subfamily, such as GDF-1, GDF-3, GDF-8, GDF-9, GDF-11 (or BMP-11), GDF-12 and GDF-14, and the members of other BMP subfamilies, such as BMP-3 (or osteogenin), BMP-9 (or GDF-2), and BMP-10 as well as BMPs 15 and 16, when produced by recombinant technology. Preferred examples of the BMPs and rBMPs useful in the pharmaceutical compositions of the present invention include natural and recombinant BMP-2, BMP-4, BMP-5, BMP-6, and BMP-7, which BMPs at present are known to be critically involved in the formation of new bone. Especially preferred are BMP-2, BMP-4, and BMP-7 having SEQ ID. NO: 1, 2, and 3, respectively. However, the scope of the invention is also intended to cover compositions containing the BMPs, whose role in the bone formation has not so far been clarified and modified rBMPs generated by eliminations or substitutions of some amino acids or by addition of some amino acids or the combination of both.

The natural BMPs useful in the present invention can be obtained for example from human bones as described by Johnson EE, et al. [Clin. Orthop. 250 (1990) 234-240] or from other mammalian bone sources as easily recognized by those skilled in the art. In the presence of triacetin the already present autologous BMP can also be sufficient.

The rBMPs useful in the present invention can be prepared by standard recombinant technology using both prokaryotic and eukaryotic expression systems in a conventional manner. In this respect, a reference is made for instance to Cerletti et al. (European Patent Application 0 433 225 A1) and Israel D.I. et al., Growth Factors 7 (1992) 139-150. The production in a prokaryotic expression system, such as in *Escherichia coli* strains or in other suitable bacterial strains, offers advantages in terms of the yield. Prokaryotic expression systems are especially suitable for the production of the rBMP monomers, which then can be dimerized for instance as described by Cerletti et al. (*supra).* On the other hand, eukaryotic expression systems, such as mammalian or insect cells, especially mammalian cells, and especially those using a protein-free medium, are advantageous in terms of the product safety. Examples of suitable mammalian cells include Chinese hamster ovarian (CHO) cells. Mammalian expression systems are especially preferable for the production of the rBMPs. However, the choice of a suitable production system is well within the knowledge of a man skilled in the art. It should also include the use of rhBMP modified in a way by addition or eliminations of amino acids that still allow signal transduction via BMP receptors to occur, especially since the mode of triacetin action seems to be downstream the BMP receptor or via the triggering of different pathways which cross talk with the BMP receptor pathways.

The glycerol mono-, di-, and triesters with carboxylic acids having 1 to 6 carbon atoms used in the pharmaceutical compositions of present invention are to have a plasticizing or solubilizing properties without having a tissue impairing effects or toxic effects in the concentrations applied. Preferably glycerol di-, and triesters with carboxylic acids having 1 to 4 carbon atoms, more preferably 2 to 3 carbon atoms, namely acetic acid and propionic acid, such as glyceryl triacetate and glyceryl diacetate, and most preferably glyceryl triacetate, are used.

The pharmaceutical composition of the invention may contain a pharmaceutically acceptable carrier. The carrier may be any suitable liquid, solid or semisolid carrier, including water, saline, buffers, such as phosphate, acetate, citrate, tartrate and similar buffers, to form a solution or a suspension, and a natural or synthetic biodegradable polymer or copolymer. In addition, the liquid carrier may contain adjuvants normally used for preparation of pharmaceutical solutions and suspensions, such as co-solvents, detergents, stabilizers, antioxidants, viscosity improving agents, preservatives, and like. Where necessary, the composition may be sterilized with any suitable sterilization method, such membrane filtration.

In one preferred embodiment of the invention the pharmaceutically acceptable carrier is a slow release system based on synthetic biogradable polymers, such as polyethylenglycol, polyglycolide, polylactides, polycaprolactones, polytrimethylenecarbonates, polyhydroxybutyrates, polyhydroxyvalerates, polydioxanones, polyorthoesters, polycarbonates, polytyrosinecarbonates, polyorthocarbonates, polyalkylene oxalates, polyalkylene succinates, poly(malic acid), poly(maleic anhydride), polypeptides, polydepsipeptides, polyvinylalcohol, polyesteramides, polyamides, polyanhydrides, polyurethanes, polyphosphazenes, polycyanoacrylates, polyfumarates, poly(amino acids), modified polysaccharides (like cellulose, starch, dextran, chitin, chitosan, etc.), modified proteins (like collagen, casein, fibrin, etc.) and their copolymers, terpolymers or combinations or mixtures or polymer blends thereof. Polyglycolide, poly(L-lactide-co-glycolide), poly(D,L-lactide-co-glycolide), poly(L-lactide), poly(D,L-lactide), poly(L-lactide-co-D,L-lactide), polycaprolactone, poly(L-lactide-co-caprolactone), poly(D,L-lactide-co-caprolactone) polytrimethylenecarbonate, poly(L-lactide-co-trimethylenecarbonate), poly(D,L-lactide-co-trimethylenecarbonate), polydioxanone and copolymers, terpolymers and polymer blends thereof are highly preferred examples of polymers. Most preferably, the copolymer of polylactide with glycolides, such as poly-DL-lactide-co-glycolide (PLGA), is used as a carrier.

Other examples of pharmaceutically acceptable carriers useful in the present invention include proteins, like collagen and fibrin, calcium phosphates (tri-calciumphospates, hydroxyapatite), bone cements, bioglass, coral minerals (Algipore®), nacre, egg shell, bovine derived hydroxyapatite (Bio-Oss®), and all osteoconductive (porous) materials.

The pharmaceutical composition of the invention may be formulated in any suitable way applicable for simultaneously administering the BMP component(s) and the glycerol mono-, di-, and triester component(s). In one preferred embodiment, the pharmaceutical composition of the invention can be prepared by mixing suitable bone formation inducing amounts of the BMP and glyceryl diacetate and optionally the pharmaceutically acceptable carrier.

Alternatively, in another embodiment of the invention a biodegradable polymer, preferably in a form of a membrane, is used as pretreated with a glycerol mono-, di-, and triester with a carboxylic acid having 1 to 6 carbon atoms, preferably with glyceryl triacetate and glyceryl diacetate, most preferably glyceryl triacetate, i.e., triacetin. The treatment comprises simply immersing the molded polymer in a solution containing triacetin for a period of time needed for softening the polymer, such as for 15 sec to 10 minutes, or to achieve a constant concentration of triacetin in the polymer and then air-drying. Then the rBMP can be introduced by heparinization [Ruppert R, Hoffmann E, Sebald W Eur. J. Biochem. 237:295-302 (1996)] of the carrier membrane. The polymer should remain flexible long enough for the insertion of the implant. Alternatively, the molded polymer is similarly pretreated with a mixture of triacetin and the rBMP, whereby the BMP attaches to the polymer by simple absorption. Alternatively, the rBMP can be applied separately but simultaneously in a different carrier, such as fibrin, collagen, polyethyleneglycol, bioclass, hydroxyapatites, calciumphosphatates and similar carrier material, such as those listed above.

In one embodiment of the invention the pharmaceutical composition is in a form of an implant, including membranes, films, plates, mesh plates, screws tabs and other formed bodies.

The pharmaceutical compositions of the invention contain a bone formation inducing amount of at least one BMP or rBMP and a bone formation inducing amount of at least one glycerol mono-, di-, and triester with a carboxylic acid having 1 to 6 carbon atoms, preferably glyceryl triacetate and glyceryl diacetate, most preferably glyceryl triacetate, i.e., triacetin. Such an amount may be for example from about 0.001 to about 10 mg, preferably from about 0.002 to about 4 mg, and more preferably from about 0.005 to about 3 mg of the BMP(s) or rBMP(s) and from about 0.001 to about 60 percent, preferably from about 0.005 to about 50 percent by weight of, for example, triacetin or diacetin. However, the bone formation inducing amounts may vary in different applications, especially in the case of the BMPs. Thus, smaller amounts may be sufficient in, for instance dental applications whereas somewhat larger amounts may be needed for the desired effect in, for instance, unilateral and multilateral spinal fusions, treatments of non-unions, treatments of osteoporosis, and in-growth of (hip) implants.

The dosage of the pharmaceutical compositions of the invention depends on the individual patients and the disorder to be treated. An exemplary single dose of a composition of the invention is within the range of about 0.001 to about 4 mg. However, a smaller dose may be sufficient in, for instance in the treatment of dental patients, whereas somewhat larger doses may be needed for the desired effect in other treatments.

The therapeutic method of the invention is based on the simultaneous use of at least one bone morphogenetic protein (BMP) and a bone formation-inducing amount of at least one glycerol mono-, di-, and triester, whereby a synergetic effect is achieved. In the method of the invention a bone formation-inducing amount of at least one bone morphogenetic protein (BMP) and a bone formation-inducing amount of a glycerol mono-, di-, and triester, optionally in a pharmaceutically acceptable carrier are simultaneously administered a subject in a need of induction of bone formation, wherein said BMP(s) and said glycerol mono-, di-, and triester, are administered in amounts providing a synergetic therapeutic effect on bone formation. For the purposes of the method of the invention, the expression "simultaneously administering" includes both simultaneous and sequential administering. Thus, in one embodiment of the method of the invention the BMP component(s) and the glycerol mono-, di-, and triester component(s) may be administered in a form of a pharmaceutical composition of the invention. In another embodiment of the method of the invention, the BMP(s) may be administered sequentially introducing a pharmaceutically acceptable carrier pretreated with a glycerol mono-, di-, and triester, such as triacetin, to the site of the treatment and then applying a suitable synergistic amount of the BMP.

The amounts of the BMP component(s) and glycerol mono-, di-, and triester component(s) used in the method vary depending on the condition to be treated and will be decided by the treating physician. Exemplary doses are as given above.

The invention is illustrated by the following examples, which are given only for illustrative purposes.

For statistical analysis, unpaired Students T-test was implemented by a commercially available software package (SSPE, Chicago, II). All values are represented as mean ± standard error of the mean.

### ANALYSIS OF DATA

The radiographs of the E1M-11®-triacetin treated animals were compared by 3 surgeons skilled on the art and compared to the radiograph of the untreated and E1M-11® treated animals.

### Cell culture technique and alkaline phosphatase determination

MC3T3-E1 cells were grown in an alpha-modified Minimum Essential Medium (Life Technologies, Inc., Grand Island, NY) containing 10 % fetal calf serum (Life Technologies, Inc.), 50µg/ml gentamycin, and 50µg/ml ascorbic acid. C3H10T1/2 cells were grown in basal medium Eagle, and 50µg/ml gentamycin. To examine the action of triacetin on both cell lines, 1x10⁵ cells per well were plated in 6-well plates and triacetin subsequently added. Medium exchange was performed after 3 days and alkaline phosphatase (ALP) was determined according to Lowry, O. et al. [J. Biol. Chem. 207 (1954) 19-37]: p-nitrophenol liberated was converted to p-nitrophenylate by adding 400 µl of 1 M NaOH, which was quantitated by measuring the absorbance at 410 nm (epsilon=17500/molxcm). Alkaline phosphatase activity was normalized to total protein and expressed as nmol nitrophenylate generated per min per mg protein.

Mineralization was determined in MC3T3-E1 cells grown in the same medium supplemented with 5mM glycerol phosphate and 1 µg/ml rhBMP-2 or 60 ng/ml of rhBMP-4 of rhBMP-7. Alizarin red-S staining was performed after 4 weeks according to Bodine, P. V. N., et al. [J. Bone Mineral Res. 11 (1996) 806-819].

### Example 1: Effect of triacetin-treated PLGA membranes on bone regeneration

To evaluate the bone regeneration, standardized defects were generated in the calvarial bone of rats. The study design included 4 rats with 8-mm artificial craniotomy defects each. The defects were either left untreated, treated with biodegradable membranes, or treated with biodegradable membranes including triacetin.

[0064] The rats were sacrificed 5 weeks after the operation and the calvarial bone was excised. Bone regeneration was determined by radiography. The evaluation was performed by eye and the judgments of 3 independent surgeons agreed on the result that triacetin improves the healing of the defect significantly. In the groups of untreated or membrane treated no healing was observed. In the group of triacetin-containing membrane treatment 3 out of 4 defects showed improved healing. Figures 1A to 1C illustrate the results showing that triacetin treated membranes enhance bone formation determined by radiography.

### Example 2: The synergistic effect of triacetin-treated PLGA membranes and rhBMP-2 on bone regeneration together

First, to verify the osteoinductive activity of triacetin an embryonic, murine, mesenchymal stem cell line C3H10T1/2 and a preosteoblastic cell line MC3T3-E1 were used and cultured as described above. C3H10T1/2 cells can differentiate into cartilage, bone or adipose cells, whereas MC3T3-E1 produces and releases autologous BMP. Alkaline phosphatase activity was used as a marker for the differentiation into the osteoblastic lineage.

As illustrated in Figure 2, the maturation of MC3T3-E1 cells increased in a concentration dependent manner with the amount of triacetin applied ranging from 0,0 to 4 mM of triacetin. Specifically, these low doses of triacetin increased the alkaline phosphatase activity by a factor of 2.0-7.0 and accelerated the maturation of preosteoblasts to osteoblasts in MC3T3-E1. This acceleration of maturation also continued for a long period of time, because the triacetin-treatment of MC3T3-E1 cells increased mineralisation performed by mature osteoblasts as determined by alizarin-S-staining (Fig. 2B). Surprisingly, this effect not only was seen for preosteoblastic cells but also for mesenchymal stem cells (C3H10T1/2). C3H10T1/2 cells treated with 2 mM and 4 mM triacetin alone showed an increase in alkaline phosphatase activity. The increase in red-staining is thus dependent on the triacetin concentration and shows that maturation determined by mineralisation is increasable by triacetin. Therefore, even triacetin alone could produce an osteoinductive effect. In the presence of rhBMP this increase was further enhanced pointing to a synergistic effect of triacetin and rhBMP even on the level of mesenchymal stem cells.

Additionally, the effect of various plasticizers/solvents regarding their osteogenetic effect was compared to that of triacetin. MC3T3-E1 cells were grown as described above and treated with increasing amounts of different solvents and the effect on the maturation of MC3T3-E1 cells determined after 6 days by alkaline phosphatase activity. The results are shown in Figure 3.

The maturation of MC3T3-E1 cells increases in a concentration dependent manner with the amount of triacetin applied ranging from 0,1 to 6 mM of triacetin. However, other solvents like methanol (MeOH), ethanol (EtOH), N, N-dimethylformamide (DMF) and dimethylsulfoxide (DMSO) showed no positive effect on the maturation of preosteoblastic cells.

To determine the effect of external BMP, C3H10T1/2 cells and MC3T3-E1 cells were plated at day one in 4 ml medium and ALP determination performed on day 6. On day 1 rhBMP was added to the cells from a stock solution of 2mg/ml rhBMP-2 in 1 mM HCl to a concentration of 1 µg/ml. triacetin to a final concentration of 2 and 4 mM was added to the cells at the same time point from a stock solution of 200 mM triacetin in respective medium. After application triacetin and rhBMP was mixed by gently swirling the entire 6 well plate. In a separate experiment, the cells were treated with triacetin only. For the calculation of the synergistic effect, the ALP activity induced by rhBMP treatment alone and the ALP activity induced by triacetintreatment alone was subtracted from the ALP activity achieved in the presence of both rhBMP and triacetin. The results are shown in Figure 4 and Figure 5A.

Figure 5A shows that in contrast to NMP, triacetin alone is able to induce alkaline phosphatase activity in the absence of rhBMP and works synergistically with rhBMP-2. The differentiation of the mesenchymal cell like cell line (C3H10T1/2) towards an osteoblastic lineage was determined by alkaline phosphatase activity. C3H10T1/2 cells were treated with triacetin in the presence or absence of 1 µg rhBMP-2/ml of the medium.

In a similar manner, the effect of external rhBMP-4 on the maturation of MC3T3-E1 cells was determined by alkaline phosphatase activity. MC3T3-E1 cells were treated with triacetin in the presence or absence of 60 ng rhBMP-4/ml of the medium. For the calculation of the synergistic effect the ALP activity induced by BMP treatment alone and the ALP activity induced by triacetin treatment alone was subtracted from the ALP activity achieved in the presence of both BMP and triacetin. The results are shown in Figure 5B.

In a similar manner the synergistic effect of triacetin and rhBMP-7 on the maturation of MC3T3-E1 cells determined by alkaline phosphatase activity. MC3T3-E1 cells were treated with triacetin in the presence or absence of 60 ng rhBMP-7/ml of the medium. For the calculation of the synergistic effect the ALP activity induced by BMP treatment alone and the ALP activity induced by NMP treatment alone was subtracted from the ALP activity achieved in the presence of both rhBMP-7 and triacetin. The results are shown in Figure 6, which clearly indicates a synergistic bone forming effect of rhBMP-7 and triacetin.

SEQUENCE LISTING<110>  University of Zurich <120>
Pharmaceutical composition<130>  2050338US<160>  3     <170>  PatentIn version
3.1<210>  1<211>  396<212>  PRT<213>  Homo sapiens<400>  1

Met Val Ala Gly Thr Arg Cys Leu Leu Ala Leu Leu Leu Pro Gln Val
1               5                   10                  15

Leu Leu Gly Gly Ala Ala Gly Leu Val Pro Glu Leu Gly Arg Arg Lys
            20                  25                  30

Phe Ala Ala Ala Ser Ser Gly Arg Pro Ser Ser Gln Pro Ser Asp Glu
        35                  40                  45

Val Leu Ser Glu Phe Glu Leu Arg Leu Leu Ser Met Phe Gly Leu Lys
    50                  55                  60

Gln Arg Pro Thr Pro Ser Arg Asp Ala Val Val Pro Pro Tyr Met Leu
65                  70                  75                  80

Asp Leu Tyr Arg Arg His Ser Gly Gln Pro Gly Ser Pro Ala Pro Asp
                85                  90                  95

His Arg Leu Glu Arg Ala Ala Ser Arg Ala Asn Thr Val Arg Ser Phe
            100                 105                 110

His His Glu Glu Ser Leu Glu Glu Leu Pro Glu Thr Ser Gly Lys Thr
        115                 120                 125

Thr Arg Arg Phe Phe Phe Asn Leu Ser Ser Ile Pro Thr Glu Glu Phe
    130                 135                 140

Ile Thr Ser Ala Glu Leu Gln Val Phe Arg Glu Gln Met Gln Asp Ala
145                 150                 155                 160

Leu Gly Asn Asn Ser Ser Phe His His Arg Ile Asn Ile Tyr Glu Ile
                165                 170                 175

Ile Lys Pro Ala Thr Ala Asn Ser Lys Phe Pro Val Thr Arg Leu Leu
            180                 185                 190

Asp Thr Arg Leu Val Asn Gln Asn Ala Ser Arg Trp Glu Ser Phe Asp
        195                 200                 205

Val Thr Pro Ala Val Met Arg Trp Thr Ala Gln Gly His Ala Asn His
    210                 215                 220

Gly Phe Val Val Glu Val Ala His Leu Glu Glu Lys Gln Gly Val Ser
225                 230                 235                 240

Lys Arg His Val Arg Ile Ser Arg Ser Leu His Gln Asp Glu His Ser
                245                 250                 255

Trp Ser Gln Ile Arg Pro Leu Leu Val Thr Phe Gly His Asp Gly Lys

            260                 265                 270

Gly His Pro Leu His Lys Arg Glu Lys Arg Gln Ala Lys His Lys Gln
        275                 280                 285

Arg Lys Arg Leu Lys Ser Ser Cys Lys Arg His Pro Leu Tyr Val Asp
    290                 295                 300

Phe Ser Asp Val Gly Trp Asn Asp Trp Ile Val Ala Pro Pro Gly Tyr
305                 310                 315                 320

His Ala Phe Tyr Cys His Gly Glu Cys Pro Phe Pro Leu Ala Asp His
                325                 330                 335

Leu Asn Ser Thr Asn His Ala Ile Val Gln Thr Leu Val Asn Ser Val
            340                 345                 350

Asn Ser Lys Ile Pro Lys Ala Cys Cys Val Pro Thr Glu Leu Ser Ala
        355                 360                 365

Ile Ser Met Leu Tyr Leu Asp Glu Asn Glu Lys Val Val Leu Lys Asn
    370                 375                 380

Tyr Gln Asp Met Val Val Glu Gly Cys Gly Cys Arg
385                 390                 395

<210> 2<211> 408<212> PRT<213> Homo sapiens<400> 2


1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175


            180                 185                 190


        195                 200                 205


    210                 215                 220


225                 230                 235                 240


                245                 250                 255


            260                 265                 270


        275                 280                 285


    290                 295                 300


305                 310                 315                 320


                325                 330                 335


            340                 345                 350


        355                 360                 365


    370                 375                 380


385                 390                 395                 400



                405

<210>  3<211>  431<212>  PRT<213>  Homo sapiens<400>  3

Met His Val Arg Ser Leu Arg Ala Ala Ala Pro His Ser Phe Val Ala
1               5                   10                  15

Leu Trp Ala Pro Leu Phe Leu Leu Arg Ser Ala Leu Ala Asp Phe Ser
            20                  25                  30

Leu Asp Asn Glu Val His Ser Ser Phe Ile His Arg Arg Leu Arg Ser
        35                  40                  45

Gln Glu Arg Arg Glu Met Gln Arg Glu Ile Leu Ser Ile Leu Gly Leu
    50                  55                  60

Pro His Arg Pro Arg Pro His Leu Gln Gly Lys His Asn Ser Ala Pro
65                  70                  75                  80

Met Phe Met Leu Asp Leu Tyr Asn Ala Met Ala Val Glu Glu Gly Gly
                85                  90                  95

Gly Pro Gly Gly Gln Gly Phe Ser Tyr Pro Tyr Lys Ala Val Phe Ser
            100                 105                 110

Thr Gln Gly Pro Pro Leu Ala Ser Leu Gln Asp Ser His Phe Leu Thr
        115                 120                 125

Asp Ala Asp Met Val Met Ser Phe Val Asn Leu Val Glu His Asp Lys
    130                 135                 140

Glu Phe Phe His Pro Arg Tyr His His Arg Glu Phe Arg Phe Asp Leu
145                 150                 155                 160

Ser Lys Ile Pro Glu Gly Glu Ala Val Thr Ala Ala Glu Phe Arg Ile
                165                 170                 175

Tyr Lys Asp Tyr Ile Arg Glu Arg Phe Asp Asn Glu Thr Phe Arg Ile
            180                 185                 190

Ser Val Tyr Gln Val Leu Gln Glu His Leu Gly Arg Glu Ser Asp Leu
        195                 200                 205

Phe Leu Leu Asp Ser Arg Thr Leu Trp Ala Ser Glu Glu Gly Trp Leu
    210                 215                 220

Val Phe Asp Ile Thr Ala Thr Ser Asn His Trp Val Val Asn Pro Arg
225                 230                 235                 240

His Asn Leu Gly Leu Gln Leu Ser Val Glu Thr Leu Asp Gly Gln Ser
                245                 250                 255

Ile Asn Pro Lys Leu Ala Gly Leu Ile Gly Arg His Gly Pro Gln Asn
            260                 265                 270

Lys Gln Pro Phe Met Val Ala Phe Phe Lys Ala Thr Glu Val His Phe
        275                 280                 285

Arg Ser Ile Arg Ser Thr Gly Ser Lys Gln Arg Ser Gln Asn Arg Ser
    290                 295                 300

Lys Thr Pro Lys Asn Gln Glu Ala Leu Arg Met Ala Asn Val Ala Glu
305                 310                 315                 320

Asn Ser Ser Ser Asp Gln Arg Gln Ala Cys Lys Lys His Glu Leu Tyr
                325                 330                 335

Val Ser Phe Arg Asp Leu Gly Trp Gln Asp Trp Ile Ile Ala Pro Glu
            340                 345                 350

Gly Tyr Ala Ala Tyr Tyr Cys Glu Gly Glu Cys Ala Phe Pro Leu Asn
        355                 360                 365

Ser Tyr Met Asn Ala Thr Asn His Ala Ile Val Gln Thr Leu Val His
    370                 375                 380

Phe Ile Asn Pro Glu Thr Val Pro Lys Pro Cys Cys Ala Pro Thr Gln
385                 390                 395                 400

Leu Asn Ala Ile Ser Val Leu Tyr Phe Asp Asp Ser Ser Asn Val Ile
                405                 410                 415

Leu Lys Lys Tyr Arg Asn Met Val Val Arg Ala Cys Gly Cys His
            420                 425                 430

## Claims

1. A pharmaceutical composition comprising at least one bone morphogenetic protein (BMP) and at least one glycerol mono-, di-, or triester with a carboxylic acid having 1 to 6 carbon atoms in a pharmaceutically acceptable carrier, wherein said BMP(s) and said glycerol mono-, di- or triester(s) are present in amounts providing a synergetic therapeutic effect on bone formation.

2. A pharmaceutical composition of claim 1, wherein said at least one glycerol mono-, di-, or triester is a glycerol di-, or triester with a carboxylic acid having 1 to 4 carbon atoms.

3. A pharmaceutical composition of claim 3, wherein said glycerol mono-, di-, or triester is selected from glyceryl triacetate and glyceryl diacetate.

4. A pharmaceutical composition of claim 3, wherein said glycerol mono-, di-, or triester is glyceryl triacetate.

5. A pharmaceutical composition of claim 1, wherein the BMP is a recombinant BMP (rBMP).

6. A pharmaceutical composition of claim 5, wherein the recombinant BMP is selected from the group consisting of rBMP-2, rBMP-3, rBMP-4, rBMP-5, rBMP-6, rBMP-7, rBMP-8, rBMP-9, rBMP10, rBMP-11, rBMP-14, rBMP-15, rBMP-16, rGDF-1, rGDF-3, rGDF-8, rGDF-9, rGDF-12, rGDF-14 and mixtures thereof.

7. A pharmaceutical composition of claim 5, wherein the recombinant BMP is selected from the group consisting of rBMP-2, rBMP-4, rBMP-5, rBMP-6, and rBMP-7 and mixtures thereof.

8. A pharmaceutical composition of claim 5, wherein the recombinant BMP is selected from the group consisting of rBMP-2, rBMP-4, rBMP-7 and mixtures thereof.

9. A pharmaceutical composition of claim 1, wherein the BMP is rBMP-2 and said glycerol mono-, di-, or triester is glyceryl triacetate.

10. A pharmaceutical composition of claim 1, wherein the pharmaceutically acceptable carrier is a biodegradable polymer.

11. A pharmaceutical composition of claim 10, wherein the biodegradable polymer is selected from the group consisting of polyglycolide, polylactides, polycaprolactones, polytrimethylenecarbonates, polyhydroxybutyrates, polyhydroxyvalerates, polydioxanones, polyorthoesters, polycarbonates, polytyrosinecarbonates, polyorthocarbonates, polyalkylene oxalates, polyalkylene succinates, poly(malic acid), poly(maleic anhydride), polypeptides, polydepsipeptides, polyvinylalcohol, polyesteramides, polyamides, polyanhydrides, polyurethanes, polyphosphazenes, polycyanoacrylates, polyfumarates, poly(amino acids), modified polysaccharides, modified proteins, and their copolymers, terpolymers and combinations and mixtures and polymer blends thereof.

12. A pharmaceutical composition of claim 10, wherein the biodegradable polymer is selected from the group consisting of polyglycolide, poly(L-lactide-co-glycolide), poly(D,L-lactide-co-glycolide), poly(L-lactide), poly(D,L-lactide), poly(L-lactide-co-D,L-lactide), polycaprolactone, poly(L-lactide-co-caprolactone), poly(D,L-lactide-co-caprolactone) polytrimethylenecarbonate, poly(L-lactide-co-trimethylenecarbonate), poly(D,L-lactide-co-trimethylenecarbonate), polydioxanone and copolymers, terpolymers and polymer blends thereof.

13. A pharmaceutical composition of claim 12, wherein the biodegradable polymer is a poly-DL-lactide-co-glycolide (PLGA).

14. A pharmaceutical composition comprising at least one recombinant bone morphogenetic protein (rBMP), at least one glycerol mono-, di-, or triester with a carboxylic acid having 1 to 4 carbon atoms, and a biodegradable polymer, wherein said rBMP(s), said glycerol mono-, di-, or triester(s) and said biodegradable polymer are present in amounts providing a synergetic therapeutic effect on bone formation.

15. A pharmaceutical composition of claim 14, wherein said at least one rBMP is selected from the group consisting of rBMP-2, rBMP-4, and rBMP-7 and mixtures thereof, said glycerol mono-, di-, or triester is glyceryl triacetate and said biodegradable polymer is a poly-DL-lactide-co-glycolide (PLGA), and wherein said recombinant BMPs, said glyceryl triacetate and said PLGA are present in amounts providing a synergetic therapeutic effect on bone formation.

16. A method of treating a subject in a need of induction of bone formation, the method comprising simultaneously administering to said subject a bone formation-inducing amount of at least one recombinant bone morphogenetic protein (rBMP) and a bone formation-inducing amount of at least one glycerol mono-, di-, or triester with a carboxylic acid having 1 to 6 carbon atoms in a pharmaceutically acceptable carrier, wherein said BMP(s) and glycerol mono-, di-, or triester(s) are administered in a pharmaceutically acceptable carrier in amounts providing a synergetic therapeutic effect on bone formation.

17. A method of claim 16, wherein said glycerol mono-, di-, or triester is glyceryl triacetate, said rBMP is selected from the group consisting of rBMP-2, rBMP-3, rBMP-4, rBMP-5, rBMP-6, rBMP-7, rBMP-8, rBMP-9, rBMP10, rBMP-11, rBMP-14, rBMP-15, rBMP-16, rGDF-1, rGDF-3, rGDF-8, rGDF-9, rGDF-12 and rGDF-14 and mixtures thereof, and said pharmaceutically acceptable carrier is a biodegradable polymer.

18. A method of claim 19, wherein said glycerol mono-, di-, or triester is glyceryl triacetate, said BMP is selected from the group consisting of rBMP-2, rBMP-4, rBMP-5, rBMP-6, and rBMP-7 and mixtures thereof, and said pharmaceutically acceptable carrier is selected from the group consisting of polyglycolide, poly(L-lactide-co-glycolide), poly(D,L-lactide-co-glycolide), poly(L-lactide), poly(D,L-lactide), poly(L-lactide-co-D,L-lactide), polycaprolactone, poly(L-lactide-co-caprolactone), poly(D,L-lactide-co-caprolactone) polytrimethylenecarbonate, poly(L-lactide-co-trimethylenecarbonate), poly(D,L-lactide-co-trimethylenecarbonate), polydioxanone and copolymers, terpolymers and polymer blends thereof.

19. A method of claim 17, wherein said glycerol mono-, di-, or triester is glyceryl triacetate, said rBMP is selected from the group consisting of rBMP-2, rBMP-4, rBMP-7, and mixtures thereof, and said pharmaceutically acceptable carrier is a poly-DL-lactide-co-glycolide (PLGA).
